# EUROPÄISCHE PATENTSCHRIFT

(11) **EP 2 424 649 B1**
(45) Veröffentlichungstag und Bekanntmachung des Hinweises auf die Patenterteilung: **07.08.2013**
(21) Anmeldenummer: 10713292.0
(22) Anmeldetag: 08.04.2010
(51) Int. Cl.: B01F 3/10, B01F 5/06, B01F 11/00, B01F 13/00, B01F 15/00, A61B 17/88

(54) **VORRICHTUNG ZUM MISCHEN UND AUSTRAGEN EINES FLUIDEN PRODUKTS SOWIE DAZUGEHÖRIGES SYSTEM**
DEVICE FOR MIXING AND DISPENSING OF A FLUIDIC PRODUCT, AND RELATED SYSTEM
DISPOSITIF POUR MELANGER ET DISPENSER D'UN PRODUIT FLUIDE, ET SYSTEME ASSOCIE

(30) Priorität: 28.04.2009 CH 660092009
(43) Veröffentlichungstag der Anmeldung: 07.03.2012
(73) Patentinhaber: Medmix Systems AG, 6343 Rotkreuz (CH)
(72) Erfinder: GRETER, Andy, 6340 Baar (CH); STÖCKLI, Rochus, 6374 Buochs (CH); KAUFMANN, Samuel, 5630 Muri (CH); KELLER, Wilhelm A., 6402 Merlischachen (CH)
(74) Vertreter: Detken, Andreas
(86) Internationale Anmeldenummer: PCT/CH2010/000091
(87) Internationale Veröffentlichungsnummer: WO 2010/124401

(56) Entgegenhaltungen:
- DE-A1- 10 242 984
- DE-A1-102005 030 510
- DE-U1-202007 007 796
- GB-A- 2 338 428
- US-A1- 2002 049 405
- US-A1- 2007 217 282

## Beschreibung

### TECHNISCHES GEBIET

Die vorliegende Erfindung betrifft eine Austragvorrichtung zum Austragen eines fluiden Produkts mit einer Mischeinrichtung zum Mischen des fluiden Produkts in der Austragvorrichtung nach dem Oberbegriff des Patentanspruchs 1.

### STAND DER TECHNIK

Austragvorrichtungen zum Austragen eines fluiden Produkts, wie die vorliegende Erfindung sie betrifft, werden bei vielerlei Anwendungen z.B. in der Medizinaltechnik eingesetzt. Eine solche Austragvorrichtung kann beispielsweise die Form einer Einzylinderspritze aufweisen, die ein zylindrisches Gehäuse mit einem Auslass am vorderen Gehäuseende und einen in dem Gehäuse aufgenommenen Kolben oder Stössel umfasst, der am gegenüberliegenden hinteren Ende des Gehäuses aus diesem herausragt. Zwischen dem Gehäuseauslass und dem Kolben ist im Inneren des Gehäuses das fluide Produkt angeordnet. Durch Vorschub des Kolbens innerhalb des Gehäuses von einer hinteren Stellung in Richtung des Auslasses zu einer vorderen Stellung, wird das fluide Produkt durch den Auslass aus dem Gehäuse ausgetragen.

Einige fluide Produkte, die mittels einer solchen Austragvorrichtung appliziert werden sollen, müssen unmittelbar vor der Anwendung verrührt oder vermischt werden. Das fluide Produkt kann dabei bereits in vollständiger Zusammensetzung in der Austragvorrichtung aufbewahrt sein oder es ist möglich, dass mehrere Komponenten des fluiden Produkts innerhalb der Austragvorrichtung zusammengeführt und vermischt werden müssen. Bei sehr zähflüssigen Fluiden ist beispielsweise ein Mischen durch Schütteln der Austragvorrichtung nicht ausreichend. Um ein ausreichendes Mischen innerhalb der Austragvorrichtung zu ermöglichen, bestehen Austragvorrichtungen, die eine Mischeinrichtung umfassen.

Für die Herstellung und Verwendung von Knochenzement ist aus der US 6,406,175 B1 beispielsweise ein Misch- und Injektionsgerät bekannt, Das Gerät umfasst einen Gehäusezylinder, der an beiden Enden durch eine Abdeckung verschlossen ist. Durch die Abdeckungen wird eine Stange geführt, die mittels eines Handgriffs in Längsrichtung innerhalb des Gehäuses verschiebbar ist. Innerhalb des Gehäuses ist an der Stange eine Mischscheibe fest angebracht, so dass durch die Längsbewegung die Mischscheibe innerhalb des Gehäuses verschoben werden kann, um ein Mischprodukt zu mischen. Innerhalb des Gehäuses ist an einer Abdeckung ein Kolbenelement angebracht. Nach Beendigung des Mischens wird die Mischscheibe in eine vordere Position geschoben und der Teil des Stabes, der am gegenüberliegenden Ende aus dem Gehäuse hervorsteht wird abgebrochen. Anschliessend wird die Mischscheibe in eine hintere Position gezogen, in der sie gegen das Kolbenelement stösst. Durch Drehung der Mischscheibe mittels des Stabs wird das Kolbenelement an der Mischscheibe befestigt. Sobald das Kolbenelement am Stab befestigt ist, kann es von der Innenseite der Gehäuseabdeckung gelöst werden und das Mischprodukt durch die beim Abbrechen des Stabes am gegenüberliegenden Gehäuseende entstandene Öffnung ausgetragen werden.

Aus der EP 1 920 738 A2 ist ein Gerät zur Verabreichung einer aushärtenden Masse bekannt. Das Gerät weist im Wesentlichen den Aufbau einer herkömmlichen Spritze mit einem Spritzengehäuse inklusive Auslass und einem darin gelagerten Ausstossglied auf. Innerhalb der Spritze ist ein Mischraum vorgesehen, in dem das Mischprodukt mittels eines Mischelements, das an dem Ausstossglied angeordnet ist, gemischt werden kann. An dem dem Auslass gegenüberliegenden Ende ist am Spritzengehäuse ein Kolbenteil gelagert, das während dem Mischvorgang relativ zum Gehäuse fest ist. Am Ende des Mischvorgangs, wird das Ausstossglied mit dem Mischelement gegen das Kolbenteil gezogen und durch Drehung des Ausstossgliedes vom Gehäuse gelöst und am Ausstossglied befestigt. Durch Vorschub des Ausstossglieds mit dem Kolbenteil kann die Mischung durch den Auslass ausgestossen werden.

Die Dokumente DE 102 42 984 und GB 2 338 428 zeigen Vorrichtungen, bei denen jeweils in einem Gehäuse, welches die Form eines äusseren Hohlzylinders aufweist, ein beweglich gelagerter innerer Hohlzylinder angeordnet ist. Der innere Hohlzylinder dient dazu, ein im Gehäuse aufgenommenes Produkt aus der Vorrichtung auszutragen. Ein Mischstab mit einem daran angebrachten Mischelement ist durch den inneren Hohlzylinder hindurchgeführt. Durch Verschieben des Mischstabes innerhalb des inneren Hohlzylinders kann das im Gehäuse aufgenommene Produkt vermischt werden.

Bei den bekannten Austrag- und Mischvorrichtungen ist es schwierig, nur kleine Mengen eines fluiden Produkts zu mischen und auszutragen. Bei Vorrichtungen mit grossem Gehäusequerschnitt ist oftmals der Hubweg zur Durchführung der Mischung mit einem Mischstab sehr kurz, so dass keine ausreichende Mischung des Fluids sichergestellt werden kann. Bei Geräten mit kleinem Durchmesser und grosser Länge der Mischkammer muss der Mischstab sehr dünn ausgebildet sein, wodurch er bei einem Mischvorgang instabil werden kann.

Es ist eine Aufgabe der vorliegenden Erfindung, eine Austragvorrichtung mit einer Mischeinrichtung zu schaffen, die ein zufriedenstellendes Mischen eines fluiden Produkts in der Vorrichtung ermöglicht, stabil in der Ausgestaltung ist und eine einfache Handhabung ermöglicht.

### DARSTELLUNG DER ERFINDUNG

Diese Aufgabe wird von der Erfindung durch eine Vorrichtung nach Anspruch 1 gelöst. Vorteilhafte Ausgestaltungen und weitere Ausführungsbeispiele sind in den abhängigen Ansprüchen beschrieben.

Nach der vorliegenden Erfindung ist eine Austragvorrichtung zum Austragen eines fluiden Produkts, wie etwa eines flüssigen oder pastösen Produkts, vorgesehen, die ein Gehäuse mit einem Auslass an einem vorderen Gehäuseende, ein Austragglied, das beweglich in dem Gehäuse gelagert ist und mit seinem hinteren Ende aus einem hinteren Gehäuseende ragt, und eine Mischeinrichtung zum Mischen des fluiden Produkts innerhalb des Gehäuses umfasst. Zwischen dem Auslass am vorderen Ende des Gehäuses und dem Austragglied ist innerhalb des Gehäuses ein Gehäusevolumen ausgebildet, in dem das fluide Produkt vorgesehen ist. Durch Vorschub des Austragglieds innerhalb des Gehäuses von einer hinteren Stellung in eine vordere Stellung auf den Gehäuseauslass zu wird das fluide Produkt durch den Auslass aus dem Gehäuse ausgetragen. Erfindungsgemäss weist die Mischeinrichtung ein Mischrohr, das zwischen dem Gehäuse und dem Austragglied beweglich im Gehäuse gelagert ist, und wenigstens ein Mischelement auf, das am vorderen Ende des Mischrohrs vorgesehen ist und innerhalb des Gehäusevolumens angeordnet ist.

Das Gehäuse ist vorzugsweise zylindrisch ausgebildet und kann z.B. die Form eines Spritzengehäuses aufweisen. Der Auslass am vorderen Ende des Gehäuses kann durch eine im Gehäuse integrierte Auslassdüse mit geringem Durchmesser gebildet werden. Es ist auch möglich, den Auslass als einfache Gehäuseöffnung auszubilden. Über dem Auslass können z.B. weitere Zubehörteile angeordnet werden, die zum Austragen für bestimmte Fluide oder bei bestimmten Applikationen besonders geeignet sind. Während einem Mischvorgang mit der Austragvorrichtung ist der Gehäuseauslass durch eine abnehmbare Abdeckung verschlossen.

Das Austragglied ist am hinteren Gehäuseende in das Gehäuse eingeführt. Vorzugsweise wird das Austragglied durch einen länglichen Zylinder gebildet, dessen vorderes Ende, das innerhalb dem Gehäuse zu liegen kommt, durch eine Frontfläche verschlossen ist. Die Frontfläche bildet eine Austragfläche, bzw. einen Kolben, zum Austragen des fluiden Produkts aus dem Gehäuse. Zwischen dem vorderen Abschluss des Gehäuses, in dem sich der Auslass befindet, der Innenumfangswand des Gehäuses und der Austragfläche des Austragglieds ist das Gehäusevolumen zur Aufnahme des fluiden Produkts ausgebildet. Durch Vorschub des Austragglieds von einer hinteren Stellung in eine vordere Stellung in Richtung auf den Auslass zu wird dieses Gehäusevolumen verkleinert und das Fluid durch den Auslass hindurch verdrängt. Nach der Erfindung ist das Austragglied zumindest teilweise innerhalb der Mischeinrichtung angeordnet. Ferner karin die Mischeinrichtung beim Vorschub des Austragglieds zum Austragen des Produkts mit verschoben werden.

Erfindungsgemäss ist das Mischrohr zwischen dem Gehäuse und dem Austragglied gelagert. Das Austragglied ist vorzugsweise innerhalb des Mischrohrs beweglich gelagert. Dabei schliesst vorzugsweise die Aussenwand des Mischrohrs an die Innenwand des Gehäuses an und die Aussenwand eines rohrförmigen Austragglieds oder eines anderen Kolbenelements schliesst an die Innenwand des Mischrohrs an. Das zylindrische Gehäuse, das Mischrohr und das Austragglied können teleskopartig relativ zueinander in Längsrichtung verschoben werden. Das Mischrohr steht mit seinem hinteren Ende aus dem hinteren Gehäuseende hervor. Das Austragglied widerum steht mit seinem hinteren Ende aus dem hinteren Ende des Mischrohres hervor. Das hintere Ende des Mischrohres, das aus dem Gehäuse hervorsteht, kann einen Griffblock aufweisen, an dem das Mischrohr ergriffen und relativ zu Gehäuse und Austragglied bewegt werden kann. Das Gehäuse kann am hinteren Ende einen Gehäuseanschlag und das Austragglied kann am hinteren Ende einen Stoppanschlag aufweisen, zwischen welchen der Griffblock hin und her verschoben werden kann. Der Gehäuseanschlag und der Stoppanschlag begrenzen somit die Verschiebebewegung des Mischrohres.

Das Mischrohr nach der vorliegenden Erfindung ragt mit seinem vorderen Ende in das Gehäusevolumen, in welchem sich das fluide Produkt befindet, hinein. An diesem Ende des Mischrohres ist das wenigstens eine Mischelement angeordnet. Das wenigstens eine Mischelement kann dabei über das Ende des Mischrohres hervorstehen, kommt jedoch vorzugsweise innerhalb der vorderen Öffnung am Rand des Mischrohres zu liegen. Das wenigstens eine Mischelement kann einstückig mit dem Mischrohr ausgebildet sein. Als Mischelemente eignen sich z.B. einzelne Schaufeln oder Leisten, die z. B. kreuzförmig oder sternförmig am Vorderende des Mischrohres derart angeordnet sind, dass ein Durchlass in das Innere des Mischrohres verbleibt. Vorzugsweise sind Mischschaufeln schräg zur Längsachse des Mischrohres angeordnet, um ein besseres Mischverhalten erzeugen zu können.

Das Mischrohr ist mit seinem Aussenumfang passgenau in dem Innenumfang des Gehäuses derart eingesetzt, dass eine Bewegung zwischen Gehäuse und Mischrohr möglich bleibt. Der Übergang zwischen der Mischrohraussenwand und der Gehäüseinnenwand ist fluiddicht ausgebildet. Hierfür kann z.B. ein O-Ring am Innenumfang des Gehäuses vorgesehen werden, der an den Aussenumfang des Mischrohres anschliesst. Es ist auch möglich, am Aussenumfang des Mischrohres eine Dichtlippe anzubringen, die in Umfangsrichtung am Gehäuseinnenumfang anliegt. Es ist darauf zu achten, dass die Dichtung ein ausreichend einfaches Verschieben des Mischrohrs im Gehäuse zu lässt. Der Übergang zwischen dem Aussenumfang des Austragglieds zum Innenumfang des Mischrohres ist ebenfalls fluiddicht ausgebildet. Vorzugsweise ist das Austragglied, rohrartig ausgebildet, so dass der Aussenumfang des Austragrohres am Innenumfang des Mischrohres zu liegen kommt. Zwischen Austragrohr und Mischrohr kann wiederum ein O-Ring oder eine Dichtlippe oder eine andere Dichtung vorgesehen werden.

Zum Mischen des fluiden Produkts in dem Gehäusevolumen wird das Austragglied in eine hintere Position gebracht und relativ zum Gehäuse in Ruhe gehalten. Das Mischrohr wird nun relativ zum Gehäuse und zum Austragglied hin und her bewegt, so dass die Mischelemente durch das Gehäusevolumen geführt werden, wobei das fluide Produkt durchmischt wird. Der Abstand des Gehäuseanschlags und des Stoppanschlags am Austragglied, zwischen welchen das Mischrohr hin und her bewegt wird, kann vorteilhafterweise derart bemessen sein, dass in einer vorderen Position die Vorderkante des wenigstens einen Mischelements an der Bodenfläche des Gehäuses anschlägt und in einer hinteren Position die rückseitige Kante des wenigstens einen Mischelements gegen die Kolbenfläche des Austragglieds anstösst. Vorzugsweise ist das Mischrohr relativ zum Gehäuse und/oder zum Austragglied drehbar gelagert. Dadurch können die Mischelemente über die Bodenfläche des Gehäuses und die Austragfläche des Austragglieds geführt werden und somit Restbestandteile des fluiden Produkts von diesen Flächen entfernen.

Bei dem Vor- und Zurückbewegen des Mischrohres innerhalb des Gehäusevolumens wird das fluide Produkt bei einer Bewegung in Richtung des Gehäuseauslasses zwischen den Mischelementen hindurch in das Innere des Mischrohres geführt. Bei einer Bewegung in Richtung des Austragglieds wird das fluide Produkt von der Austragfläche des Austragglieds an den Mischelementen vorbei aus dem Mischrohr in das Gehäusevolumen ausgestossen.

Bei einer Austragvorrichtung gemäss der vorliegenden Erfindung, ist es möglich, auch kleine Volumina eines fluiden Produkts zuverlässig zu durchmischen, da das Mischrohr durch die Rohrkonstruktion stabil ausgebildet ist und die Innenumfangswand des Gehäuses eine Führung für das Mischrohr bilden kann. Das Mischrohr kann daher kräftig innerhalb des Gehäuses bewegt werden, um eine starke Verwirbelung des fluiden Produkts im Gehäuse zu erzeugen, ohne dass ein Verwinden oder Abknicken erfolgt.

Bei einer bevorzugten Ausführungsform der Austragvorrichtung nach der vorliegenden Erfindung ist eine Arretiereinrichtung zur lösbaren Arretierung des Austragglieds in einer hinteren Stellung am Gehäuse vorgesehen. Die Arretiereinrichtung stellt sicher, dass das Austragglied bei dem Mischvorgang relativ zum Gehäuse feststeht und nicht gemeinsam mit der Bewegung des Mischrohres bewegt wird. Nach Beendigung des Mischvorganges, kann die Arretierung des Austragglieds gelöst werden und das Austragglied relativ zum Gehäuse zum Austragen des fluiden Produkts vorgeschoben werden.

Bei einer ersten Variante einer Arretiereinrichtung ist wenigstens ein Gleitarm am Austragglied und wenigstens ein Gleitkanal am Gehäuse vorgesehen in welchen der Gleitarm gleitend einführbar ist. Vorzugsweise sind zwei Gleitarme parallel neben dem Austragglied angeordnet. Beispielsweise können die Gleitarme von einer gemeinsamen Endplatte, die am hinteren Ende des Austragglieds angeordnet ist abragen. Die Endplatte kann zudem als Stoppanschlag für den Griffblock des Mischrohres dienen. Für jeden der beiden Gleitarme ist ein Gleitkanal am Gehäuse vorgesehen. Die Gleitkanäle können z.B. neben dem zylindrischen Teil des Gehäuses im Gehäusematerial ausgeformt sein. Der Gleitkanal kann auch als gesondertes Element am Gehäuse befestigt sein. In zusammengesetztem Zustand des Austragglieds und des Gehäuses ragt das vorzugsweise rohrförmige Austragglied in das zylindrisch ausgebildete Gehäuse hinein, während die parallel dazu verlaufenden Gleitarme in die Gleitkanäle hineinragen. Die Gleitarme in den Gleitkanälen bilden somit eine Führung für das Austragglied beim Vor- und Zurückbewegen innerhalb des Gehäuses unabhängig von einer Arretierfunktion.

Zur Arretierung des Austragglieds am Gehäuse können an den Gleitarmen Rastmittel vorgesehen sein, die mit Gegenrastmittel am oder im Gleitkanal zusammen wirken und ein lösbares Verrasten ermöglichen. Das Rastmittel kann z.B. durch wenigstens einen flexibel vom Gleitarm abstehenden Rastarm, gebildet werden. Das Gegenrastmittel kann durch wenigstens eine Rastöffnung in der Gleitfläche des Gleitkanals gegeben sein. Die Rastarme und Rastöffnungen sind derart positioniert, dass die Rastarme in die Rastöffnungen eingreifen, wenn das Austragglieds in einer hinteren Stellung relativ zum Gehäuse ist.

Bei einer anderen Variante wird die Arretiereinrichtung durch wenigstens einen Rastflügel an einem von Gehäuse und Austragglied und wenigstens eine Rastnut am anderen von Gehäuse und Austragglied gegeben. Vorzugsweise sind zwei Rastflügel am Gehäuse und zwei Rastnuten am Austragglied vorgesehen. Die Rastflügel ragen gegenüberliegend radial vom Aussenumfang des Gehäuses ab. Die Rastnuten sind an parallel zum Austragglied seitlich versetzten Auslegern angeordnet. Bei einer Verdrehung des Austraggliedes relativ zum Gehäuse kommen die Rastflügel innerhalb der Rastnuten zu liegen. Ein weiteres Verdrehen wird dadurch verunmöglicht, dass die Rastflügel gegen Anschläge innerhalb der Rastnuten anschlagen. Durch ein Rückdrehen wird die Arretierung gelöst. Sobald die Rastflügel in die Rastnuten eingreifen, ist eine Längsbewegung des Austraggliedes relativ zum Gehäuse nicht mehr möglich. Vorzugsweise kommt der Griffblock des Mischrohres zwischen den parallel verlaufenden Auslegern des Austraggliedes zu liegen. Besonders bevorzugt, gleitet die Aussenfläche des Griffblocks entlang der Innenfläche der Ausleger.

In einer weiteren Ausführungsform einer Austragvorrichtung nach der vorliegenden Erfindung ist eine Blockiereinrichtung zur Blockierung einer Bewegung des Austragglieds relativ zum Gehäuse in der hinteren Stellung des Autragglieds entgegen der Vorschubrichtung vorgesehen. Die Blockiereinrichtung verhindert ein versehentliches vollständiges Herausziehen des Austragglieds aus dem Gehäuse. Das Mischrohr ist vorzugsweise mit einem hinteren Anschlag, wie etwa dem Griffblock, ausgebildet, der bei einer Bewegung entgegen der Vorschubrichtung relativ zum Austragglied gegen einen Stoppanschlag am hinteren Ende des Austragglieds stösst, so dass diese Bewegung blockiert wird. Aufgrund der Blockiereinrichtung für eine Bewegung entgegen der Vorschubrichtung für das Austragglied in der hinteren Stellung des Austragglieds, ist durch den Stoppanschlag auch das Mischrohr an einem vollständigen Herausziehen aus dem Gehäuse gehindert.

Die Blockiereinrichtung kann z.B. an wenigstens einem Gleitarm mit Rastarm und wenigstens einem Gleitkanal mit Rastöffnung ausgebildet sein, wie sie vorher beschrieben wurden. Zur Blockierung des Austraggliedes stösst ein in die Rastöffnung eingerasteter Rastarm mit seinem Ende gegen einen Rand der Rastöffnung, so dass die Rastöffnung einen Anschlag für das Rastarmende bildet.

In der Vorschubrichtung ist eine Bewegung des Austragglieds relativ zum Gehäuse möglich, in dem der flexibel ausgebildete Rastarm am Rand der Rastöffnung entlang gleitet und dadurch flexibel radial nach innen gebogen wird. Die Fläche des Rastarms, die am Rand der Rastöffnung des Gehäuses entlang gleitet, kann einen Rastnocken aufweisen, welcher der Vorschubbewegung des Austragglieds einen Widerstand entgegensetzt. Der Rastnocken ermöglicht die Arretierung des Austragglieds in der hinteren Stellung relativ zum Gehäuse. Um das Austragglied in Vorschubrichtung zu bewegen, muss zunächst ein erhöhter Kraftaufwand erfolgen, um den Widerstand des Rastnockens zu überwinden. Sobald der Widerstand überwunden wurde, ist der Rastarm flexibel nach innen gebogen und das Austragglied kann in Vorschubrichtung bewegt werden. Durch den hinteren Anschlag des Mischrohrs am Stoppanschlag des Austragglieds wird das Mischrohr bei einer Vorschubbewegung des Austragglieds gemeinsam mit dem Austragglied bewegt und in das Gehäuse eingeschoben.

Ferner kann bei einer Austragvorrichtung nach der vorliegenden Erfindung eine Führungseinrichtung zur Führung des Mischrohrs bei einer Verschiebung entlang dem Austragglied vorgesehen sein. Eine Führungseinrichtung kann beispielsweise durch wenigstens einen Gleitarm am Austragglied, der parallel zum Austragglied verläuft, und wenigstens eine Gleitdurchführung an einem Griffblock des Mischrohrs ausgebildet sein. Der Gleitarm des Austragglieds ist gleitend in der Gleitdurchführung aufgenommen. Der Gleitarm am Austragglied dient zur Stabilisierung des Mischrohrs bei seiner Bewegung relativ zum Austragglied und zum Gehäuse. Wie oben bereits beschrieben, kann der Gleitarm des Austragglieds in einen Gleitkanal am Gehäuse münden, wodurch auch der Gleitarm stabilisiert wird.

Um eine Drehbewegung des Mischrohres um das Austragglied zu ermöglichen, kann die Gleitdurchführung am Griffblock schlitzartig in Kreissegmentform ausgebildet sein, so dass der Gleitarm innerhalb des Schlitzes von einem Ende zum anderen Ende gedreht werden kann. Das Kreissegment der Gleitdurchführung im Griffblock definiert daher den möglichen Drehwinkel des Mischrohrs um das Austragglied.

Bei einer anderen Austragvorrichtung nach der vorliegenden Erfindung kann das Austragglieds am hinteren Ende eine Endplatte aufweisen von der zu beiden Seiten des Austragglieds parallel zum Austragglied verlaufende Ausleger oder Stege vorgesehen sind. Die Ausleger schliessen somit zwischen sich das Austragrohr des Austragglieds ein. Am vorderen Ende der Ausleger befinden sich Rastnuten. Am hinteren Ende des Gehäuses sind zwei zu gegenüberliegenden Seiten abragende Rastflügel vorgesehen. Beim Vor- und Zurückschieben des Austragglieds in das Gehäuse kommen die Rastflügel zwischen den Auslegern an dem Austragglied zu liegen. In der hinteren Stellung des Austragglieds enden die Ausleger hinter den Rastflügeln des Gehäuses, so dass das Gehäuse relativ zu den Stegen verdreht werden kann. Die Rastnuten stehen jedoch derart von der Vorderfläche der Ausleger ab, dass bei einer Drehung des Gehäuses die Rastflügel in diese Rastnuten einrasten. Die Rastflügel werden nun in axialer Richtung von den Rastnuten gehalten, so dass das Austragglied nicht relativ zum Gehäuse in Längsrichtung bewegt werden kann. Die Ausleger des Austragglieds mit den Rastnuten und die Rastflügel am Gehäuse bilden somit miteinander eine Arretiereinrichtung zur lösbaren Arretierung des Austragglieds in der hinteren Stellung am Gehäuse.

Der Griffblock am hinteren Ende des Mischrohrs einer Mischeinrichtung der Austragvorrichtung kann zwischen den Stegen des Austragglieds zu liegen kommen und liegt mit seinen Seiten vorzugsweise an den Innenseiten der Ausleger an. Die Innenseiten der Ausleger bilden somit eine Führungsfläche für den Griffblock des Mischrohrs. Das Mischrohr kann durch Ergreifen des Griffblocks zwischen der Abschlussplatte des Austragglieds und den Rastflügel in Längsrichtung des Austragglieds hin- und herbewegt werden. Dabei dienen die Abschlussplatte des Austragglieds und die Rastflügel des Gehäuses als Anschläge für die Bewegung des Mischrohrs.

Nach Beendigung des Mischvorgangs, kann das Gehäuse wieder aus den Rastnuten herausgedreht werden, bis die Rastflügel im Zwischenraum der Ausleger zu liegen kommen. In dieser Stellung kann das Austragglied relativ zum Gehäuse vorgeschoben werden und die Mischung ausgetragen werden.

Eine Austragvorrichtung nach der vorliegenden Erfindung kann bereits mit einem fluiden Produkt gefüllt sein, das jedoch vor einer Applikation mit der Austragvorrichtung gründlich durchmischt werden soll. Es ist jedoch auch möglich, eine Austragvorrichtung nach der vorliegenden Erfindung erst vor der Anwendung mit einem fluiden Produkt zu befüllen. Dabei ist es auch möglich, mehrere verschiedene Komponenten in die Austragvorrichtung zu füllen. Grundsätzlich ist es auch möglich, dass eine der Komponenten bereits in der Austragvorrichtung vorgesehen ist, wie beispielsweise eine Pulverkomponente, und eine weitere Komponente erst kurz vor der Anwendung in die Austragvorrichtung gefüllt wird, um mit der bereits in der Austragvorrichtung befindlichen Komponente vermischt zu werden. Zum Befüllen der Austragvorrichtung ist es möglich, an den Auslass des Gehäuses z.B. eine Spritze anzuschliessen, mit welcher ein Produkt oder eine Komponente in das Gehäuse eingespritzt werden kann. Alternativ ist es auch möglich, den Auslass des Gehäuses an ein Fluidreservoir anzuschliessen und das Fluid mittels der Austragvorrichtung in das Gehäuse einzusaugen. Hierfür wird das Austragglied gemeinsam mit dem Mischrohr von einer vorderen Position in eine hintere Position relativ zum Gehäuse gezogen, so dass eine Sogwirkung am Auslass des Gehäuses entsteht und das Fluid in die Austragvorrichtung eingesaugt wird.

Gemäss der Erfindung ist daher ein System aus einer Austragvorrichtung und einer Ladevorrichtung vorgesehen, mit der die Austragvorrichtung geladen werden kann. Vorzugsweise befindet sich in dem Gehäusevolumen der Austragvorrichtung bereits eine Komponente einer fluiden Mischung und mit der Ladevorrichtung wird eine zweite Komponente der fluiden Mischung in die Austragvorrichtung eingebracht. Mit Hilfe der Mischeinrichtung der Austragvorrichtung können die erste und die zweite Komponente innerhalb der Austragvorrichtung gemischt werden.

Eine Austragvorrichtung nach der vorliegenden Erfindung, ermöglicht eine unkomplizierte Anwendung auch kleiner Fluidmengen und eine zuverlässige Vermischung eines fluiden Produkts innerhalb der Austragvorrichtung. Ein Abknicken oder sich Verwinden der Mischeinrichtung innerhalb des Gehäuses der Austraganordnung, wodurch die Funktionalität der Austragvorrichtung negativ beeinträchtigt wird, ist nicht möglich, da die Mischeinrichtung stabil innerhalb der Austragvorrichtung vorgesehen ist und einfach zu betätigen ist.

### KURZE BESCHREIBUNG DER ZEICHNUNGEN

Bevorzugte Ausführungsformen der Erfindung werden im Folgenden anhang der Zeichnungen dargestellt, die lediglich zur Erläuterung dienen und nicht einschränkend auszulegen sind. In den Zeichnungen offenbar werdende Merkmale der Erfindung sollen einzeln und in jeder Kombination als zur Offenbarung der Erfindung gehörend betrachtet werden. In den Zeichnungen zeigen:
- Fig. 1:: Explosionsdarstellung einer ersten Ausführungsform einer Austragvorrichtung nach der vorliegenden Erfindung,
- Fig. 2a:: Längsschnitt durch die erste Ausführungsform einer Austragvorrichtung nach der vorliegenden Erfindung in einer Ausgangsposition,
- Fig. 2b:: Längsschnitt durch die einer Austragvorrichtung nach Figur 2a mit alternativer Dichtung,
- Fig. 3:: Längsschnitt der Austragsvorrichtung bei einem Mischvorgang,
- Fig. 4:: Längsschnitt der Austragvorrichtung beim Austragen eines Fluids,
- Fig. 5:: Längsschnitt durch eine Austragvorrichtung mit einer Ladevorrichtung,
- Fig. 6:: dreidimensionale Darstellung einer zweiten Ausführungsform einer Austragvorrichtung nach der vorliegenden Erfindung in einer entriegelten Position und
- Fig. 7:: Austragvorrichtung nach Figur 6 in einer verriegelten Position.

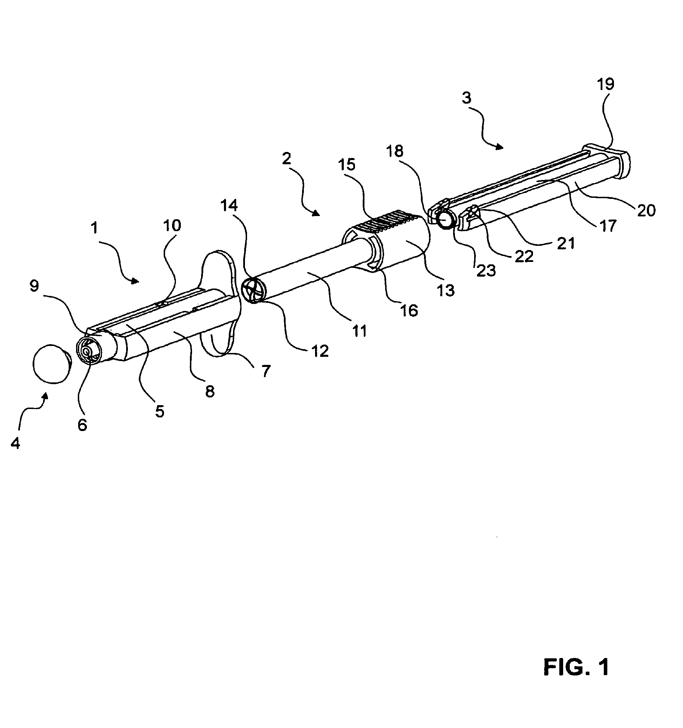

In Figur 1 ist eine Explosionsdarstellung der einzelnen Bauteile einer ersten Ausführungsform einer Austragvorrichtung nach der vorliegenden Erfindung gezeigt. Die Austragvorrichtung umfasst ein Gehäuse 1, eine Mischeinrichtung 2, ein Austragglied 3 und eine Abdeckkappe 4. Das Gehäuse 1 weist einen länglichen Gehäusezylinder 5 mit einem Auslass 6 am vorderen Ende und seitlich abragende Halteflügel 7 am hinteren Ende auf. Parallel zum Gehäusezylinder 5 erstrecken sich auf gegenüberliegenden Seiten zwei Leisten 8, die zumindest am vorderen und hinteren Ende des Gehäusezylinders 5 befestigt sind. Innerhalb der Leisten 8 ist jeweils ein Gleitkanal angeordnet, der am hinteren Ende der Leiste geöffnet ist und sich bis zum vorderen Ende erstreckt. Der Auslass 6 wird von einer Hülse 9 umgeben, die an ihrem Innenumfang ein Gewinde aufweist und als Befestigungsmittel für Zubehörteile dient, die mit der Austragvorrichtung verbunden werden sollen. Grundsätzlich sind auch andere Befestigungseinrichtungen möglich, wie etwa Steck- oder Klemmverbindungen. Entlang der Längsseite der Leisten 8 weisen die Gleitkanäle Rastöffnungen 10 auf, die sich an einem hinteren Bereich der Leisten befinden.

Die Mischeinrichtung 2 umfasst ein längliches Mischrohr 11, an dessen vorderen Ende Mischelemente 12 und an dessen hinterem Ende ein Griffblock 13 angeordnet sind. Der Aussenumfang des Mischrohrs 11 ist auf den Innenumfang des Gehäusezylinders 5 derart abgestimmt, dass das Mischrohr am Gehäusezylinder anliegt, jedoch relativ zum Gehäusezylinder verschieblich und drehbar ist. Die Mischelemente 12 am vorderen Ende des Mischrohres befinden sich innerhalb der Öffnung des Mischrohres. Am Aussenumfang des Mischrohres 11 ist eine Dichtlippe 14 vorgesehen, welche den Übergang zwischen dem Aussenumfang des Mischrohres und dem Innenumfang des Gehäusezylinders 5 fluiddicht abdichtet. Die Dichtlippe ist derart flexibel ausgebildet, dass sie an dem Innenumfang des Gehäusezylinders 5 entlang gleitet und nur wenig Reibwiderstand bei einer Verschiebebewegung zwischen dem Gehäusezylinder 5 und dem Mischohr 11 bietet. Der Griffblock 13 ist trommelartig ausgebildet und weist an zwei gegenüberliegenden Seiten Grifflächen 15 auf. Der Durchmesser des Griffblocks 13 ist grösser als der Durchmesser des Mischrohres 11, so dass der Griffblock 13 einen Verschiebeanschlag beim Einschieben des Mischrohres 11 in den Gehäusezylinder 5 am hinteren Ende des Gehäusezylinders 5, bzw. an den Halteflügeln 7 bildet. Das Mischrohr 11 verläuft vollständig durch den Griffblock und ist am hinteren Ende geöffnet. Seitlich neben dem Volumen des Mischrohres sind in Längsrichtung im Griffblock Gleitdurchgänge 16 an zwei sich gegenüberliegenden Seiten vorgesehen. Die Gleitdurchgänge 16 sind kreissegmentartig um das Mischrohr 11 angeordnet und erstrecken sich von der Vorderseite bis zur Rückseite des Griffblocks. Sofern das Mischrohr 11 in den Gehäusezylinder 5 eingeführt ist, können die Gleitdurchgänge 16 mit den Gleitkanälen in den Leisten 8 des Gehäuses 1 fluchten.

Das Austragglied 3 weist ein in Längsrichtung verlaufendes Austragrohr 17 auf, das am vorderen Ende durch eine Austragfläche 18 abgeschlossen ist. Am hinteren Ende ist an dem Austragrohr 17 eine Druckplatte 19 angeordnet, die sich quer zum Austragrohr erstreckt. Zu beiden Seiten des Austragrohrs 17 erstrecken sich parallel verlaufende Gleitarme 20, die stegartig von der Druckplatte 19 nach vorne abragen und sich im Wesentlichen bis zum vorderen Ende des Austragrohres 17 erstrecken. Am vorderen Ende der Gleitarme 20 ist an einer Längsseite der Gleitarme 20 jeweils ein flexibel vorspannbarer Rastarm 21 angeordnet. Der Rastarm 21 steht im entspannten Zustand über die Längsfläche des Gleitarms 20 über und kann flexibel nach innen gedrückt werden, so das er nicht über die Fläche der Längsseite des Gleitarms 20 hervorsteht. Auf der Aussenseite des Rastarms 21 ist seitlich ein Rastnocken 22 angeordnet.

Der Aussendurchmesser des Austragrohrs 17 ist passgenau auf den Innendurchmesser des Mischrohres 11 abgestimmt, so dass die Aussenoberfläche des Austragrohrs 17 an der Innenoberfläche des Mischrohrs 11 zu liegen kommt und das Austragrohr 17 innerhalb des Mischrohrs 11 gleitend geführt ist. Der Übergang zwischen dem Aussenumfang des Austragrohrs 17 und dem Innenumfang des Mischrohrs 11 ist fluiddicht abgedichtet mit einer Dichtlippe 23 am vorderen Ende des Austragrohrs 17. Ist das Austragrohr 17 in das Mischrohr 11 eingeführt, erstrecken sich die Gleitarme 20 durch die Gleitdurchgänge 16 des Griffblocks 13 am Mischrohr 11 bis in die Gleitkanäle in den Leisten 8 am Gehäuse 1.

Der Gehäusezylinder 5, das Mischrohr 11 und das Austragrohr 17 sind teleskopartig in Längsrichtung zueinander verschiebbar. Das Austragrohr 17 ist gegenüber dem Gehäusezylinder 5 verdreh gesichert, da die Gleitarme 20 in die Gleitkanäle der Leisten 8 des Gehäuses eingreifen und somit ein Verdrehen des Austragglieds 3 gegenüber dem Gehäuse 1 verhindern. Das zwischen dem Gehäusezylinder 5 und dem Austragrohr 17 angeordnete Mischrohr 11 ist relativ zum Gehäusezylinder 5 und zum Austragrohr 17 verdrehbar, da sich die Gleitdurchgänge 16 kreisförmig um das Mischrohr 11 erstrecken und eine grössere Länge in Umfangsrichtung aufweisen als die Breite der Gleitarme 20 beträgt. Die Gleitarme. 20 können daher innerhalb der Gleitdurchgänge 16 in Umfangsrichtung um das Mischrohr von einem Ende des Gleitdurchgangs zum anderen Ende bewegt werden. Die Enden der Gleitdurchgänge 16 bilden somit einen Anschlag für die Drehbewegung des Mischrohres 11 relativ zum Austragrohr 17.

Die Kappe 4 ist zylindrisch ausgebildet und weist auf ihrer Aussenseite ein Aussengewinde auf, welches mit dem Innengewinde der Hülse 9 am Gehäuse 1 zusammenwirken kann. Die Kappe 4 kann somit sicher über dem Auslass 6 des Gehäuses 1 angebracht werden.

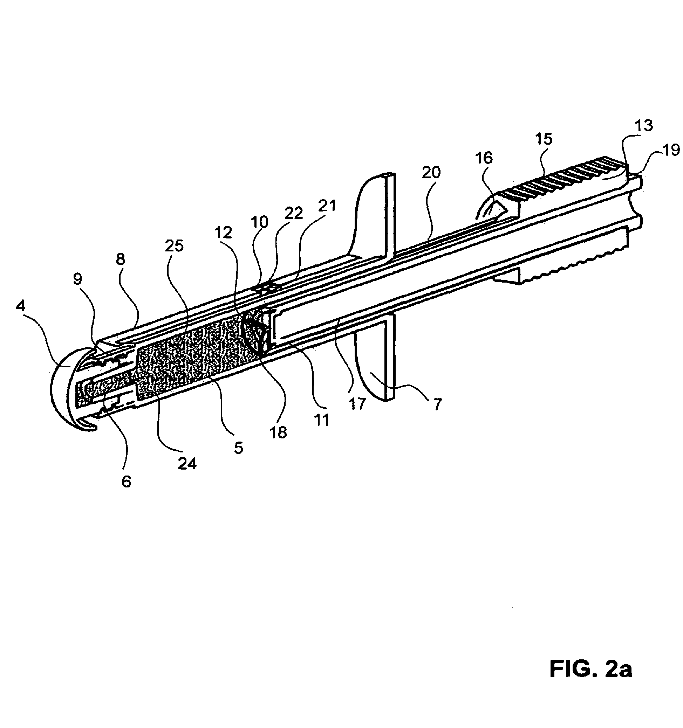

In Figur 2a ist die Austragvorrichtung nach Figur 1 in zusammengesetztem Zustand gezeigt. Die Abdeckkappe 4 ist über dem Auslass 6 in die Hülse 9 eingeschraubt. Das Austragglied 3 ist mit dem Austragrohr 17 vollständig in das Mischrohr 11 der Mischeinrichtung 2 eingeschoben, so dass die Austragfläche 18 gegen die Mischelemente 12 stösst. Die hintere Fläche des Griffblöcks 13 stösst dabei gegen die Druckplatte 19 am hinteren Ende des Austragrohrs 17. Das Austragglied 3 mit der Mischeinrichtung 2 ist teilweise in das Gehäuse 1 eingesetzt. Hierfür ist das Mischrohr 11 mit dem darin befindlichen Austragrohr 17 in den Gehäusezylinder 5 teilweise eingeschoben. Die Gleitarme 20 ragen dabei soweit in die Gleitkanäle der Leisten 8 am Gehäuse 1 hinein, bis die Rastarme 21 in die Rastöffnungen 10 eingreifen. Dabei stösst das hintere Ende eines Rastarms 21 gegen den Rand der Rastöffnung 10, so dass der Gleitarm 20 nicht nach hinten aus dem Gleitkanal gezogen werden kann. Dadurch wird ein Herausziehen des Mischrohrs 11 und des Austragrohrs 17 aus dem Gehäusezylinder 5 blockiert. Der Rastnocken 22 kommt dabei vor dem gegenüberliegenden Rand des Mischrohres zu liegen. Der Rastnocken 22 und das Ende des Rastarms 21, die beide am Rand der Rastöffnung 10 anstossen bilden somit eine Arretierung des Austragglieds 3 innerhalb des Gehäuses 1. Im Inneren des Gehäusezylinders 5 zwischen einem Boden 24 des Zylinders, in dem sich der Auslass 6 befindet, und der Austragfläche 18 am Austragrohr 17 befindet sich ein Gehäusevolumen 25, in dem ein fluides Produkt oder auch nur eine Produktkomponente, wie z. B. ein Pulver, aufgenommen ist.

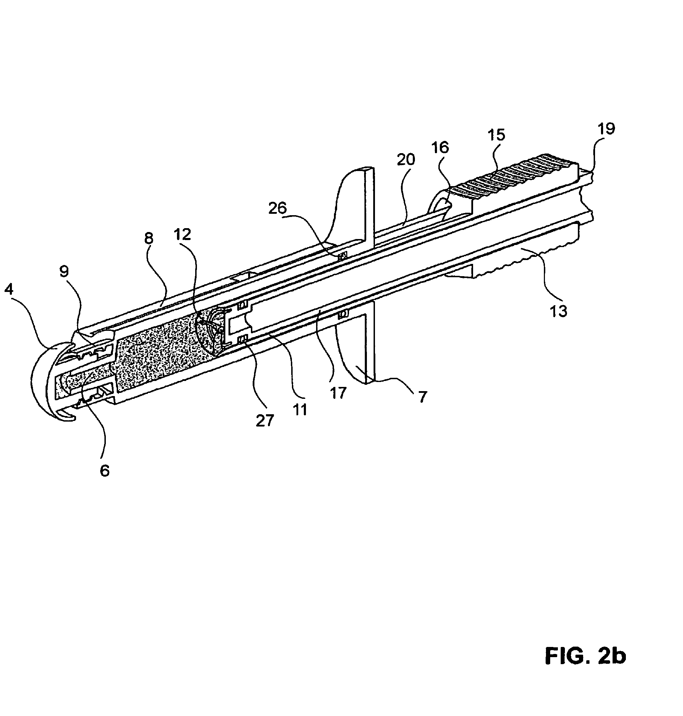

In Figur 2b ist eine Variante der Austragvorrichtung gemäss Figur 1 gezeigt, bei der an Stelle von Dichtlippen zur Herstellung eines fluiddichten Übergangs zwischen Gehäuse 1 und Mischrohr 11 und zwischen Mischrohr 11 und Austragrohr 17 O-Ringe verwendet werden, z. B. aus Gummi hergestellt sind. Ein erster O-Ring 26 ist zwischen Gehäuse 1 und Mischrohr 11 in einer Umfangsnut in der Innenwand des Gehäuses vorgesehen und liegt an der Aussenwand des Mischrohrs 11 an. Ein zweiter O-Ring 27 ist zwischen Mischrohr 11 und Austragrohr 17 in einer Umfangsnut im Aussenumfang des Austragrohres 17 angeordnet und liegt an der Innenwand des Mischrohrs 11 an. Die O-Ringe 26 und 27 schliessen das Gehäusevolumen 25 fluiddicht ab, erlauben aber ein einfaches Verschieben von Mischrohr 11 und Austragrohr 17 relativ zum Gehäuse 1.

Der Zustand der Austragvorrichtung gemäss den Figuren 2a und 2b entspricht z.B. einem Lager- oder Auslieferungszustand, bevor eine Anwendung mit der Austragvorrichtung durchgeführt wird.

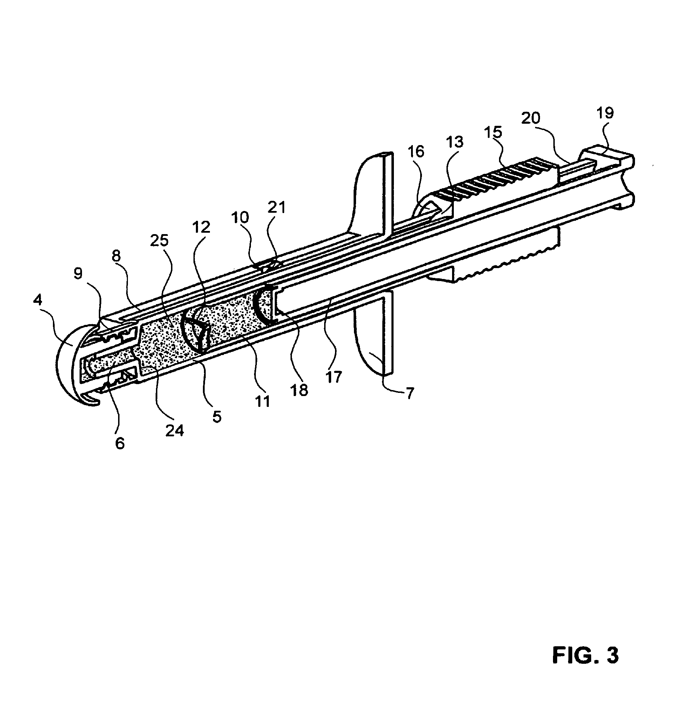

In Figur 3 ist die Austragvorrichtung bei einem Mischvorgang des, fluiden Produkts gezeigt. Zum Mischen des fluiden Produkts in dem Gehäusevolumen 25 wird das Mischrohr 11 an der Grifffläche 15 am Griffblock 13 mit einer Hand ergriffen und mit der anderen Hand wird z.B. das Gehäuse 1 gehalten. Das Mischrohr 11 wird mittels dem Griffblock 13 zwischen dem vorderen Anschlag an den Halteflügeln 7 des Gehäuses und dem hinteren Anschlag an der Druckplatte 19 des Austragglieds vor und zurück bewegt. Dabei wird das Mischrohr 11 zwischen dem Gehäusezylinder 5 und dem Austragrohr 17 innerhalb dem Gehäusezylinder 5 verschoben. Das Austragrohr 17 bleibt relativ zum Gehäusezylinder fest, da der Rastarm 21 des Gleitarms 20 innerhalb dem Gleitkanal der Leisten 8 in die Rastöffnung 10 eingreift. Beim Verschieben des Mischrohrs 11 werden die Mischelemente 12 zwischen dem Boden 24 des Gehäusezylinders 5 und der Austragfläche 18 den Austragrohrs 17 hin- und herbewegt. Die Mischelemente 12 verursachen eine Verwirbelung des fluiden Produkts innerhalb dem Gehäusevolumens 25. Bei einem Vorschieben des Mischrohrs 11 wird dabei das fluide Produkt im Inneren des Mischrohres 11 aufgenommen und bei einem Zurückschieben des Mischrohres 11 mittels der Austragfläche 18 wieder aus dem Mischrohr ausgestossen.

Es ist möglich, dass am Boden 24 und an der Austragfläche 18 Überreste oder Partikel des fluiden Produkts haften bleiben: Um diese zu lösen, kann das Mischrohr innerhalb dem Gehäusezylinder 5 verdreht werden. Dazu wird das Mischrohr in einer vordersten Position, in welcher die Mischelemente 12 am Boden 24 des Gehäusezylinders 5 anliegen, mittels dem Griffblock 13 um das Austragrohr 17 innerhalb der Gleitdurchgänge 16 gedreht. Bei dieser Drehung schaben die Mischelemente 12 über die Oberfläche des Bodens 24 und können anhaftende Teile des fluiden Produkts lösen. In der hintersten Position des Mischrohres 11, in welcher der Griffblock 13 gegen die Druckplatte 19 anschlägt, liegen die Mischelemente 12 an der Austragfläche 18 des Austragrohrs 17 an. In dieser Position wird wiederum das Mischrohr 11 mittels des Griffblocks 13 um das Austragrohr 17 herum verdreht, so dass die Mischelemente 12 über die Austragfläche 18 schaben und haftendes Material des fluiden Produkts lösen.

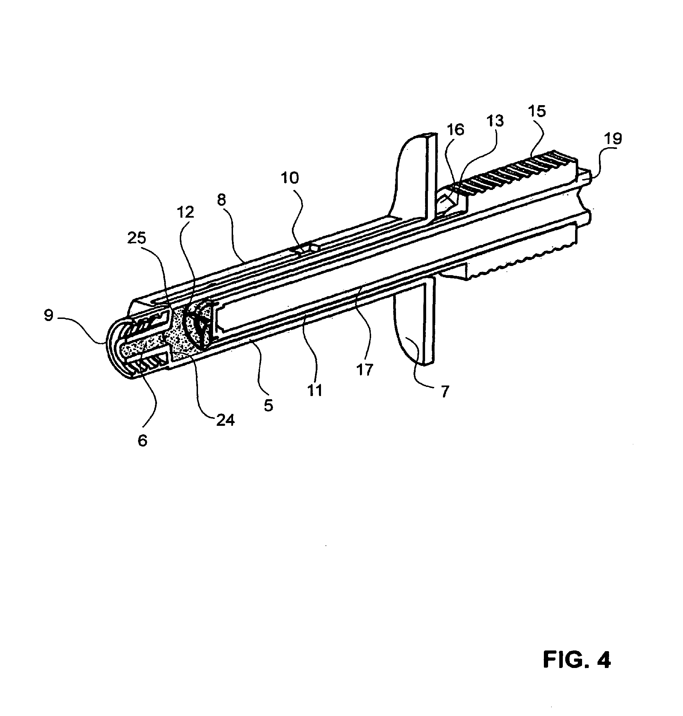

Nach Beendigung des Mischvorgangs kann das fluide Produkt aus der Austragvorrichtung ausgetragen werden. Wie in Figur 4 gezeigt ist, wird zum Austragen des Produkts die Kappe 4 von dem Auslass 6 entfernt. Das Austragrohr 17 wird relativ zum Gehäusezylinder 5 in Vorschubrichtung in den Gehäusezylinder 5 eingeschoben. Hierfür wird z.B. das Gehäuse 1 zwischen Zeige- und Mittelfinger genommen, so dass die Halteflügel 7 an den Fingern anliegen und der Daumen greift an der Druckplatte 19 an. Beim Zusammendrücken der Finger, um das Austragrohr 17 in den Gehäusezylinder 5 einzuschieben, muss zunächst ein Anfangswiderstand überwunden werden, der durch das Anstossen des Rastnocken 22 am Rand der Rastöffnung 10 erzeugt wird. Durch den Druck in Längsrichtung auf das Austragrohr 17 wird vom Rand der Rastöffnung 10 eine Kraft auf den Rastarm 21 erzeugt, welche diesen nach innen zu biegen sucht. Sobald der Anfangswiderstand des Rastnockens 22 überwunden ist, versinkt der Rastarm 21 innerhalb des Gehäusezylinders 5, so dass zum weiteren Vorschub des Austragrohrs 17 eine geringere Kraft erforderlich ist. Beim Vorschub des Austragrohrs 17 presst die Austragfläche 18 gegen das fluide Produkt innerhalb des Gehäusevolumens 25 und treibt dieses durch den Auslass 6 aus dem Gehäusezylinder 5 aus.

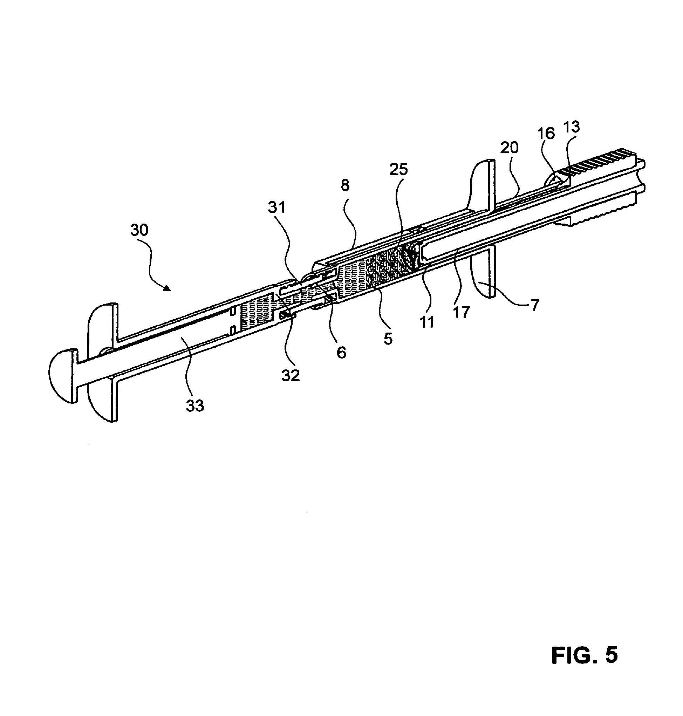

In Figur 5 ist ein System aus einer erfindungsgemässen Austragvorrichtung und einer Ladevorrichtung 30 in Form einer Spritze gezeigt. Die Spritze wird mittels eines Kupplungsstücks 31 an den Auslass 6 der Austragvorrichtung angekoppelt Dabei entsteht eine Fluidverbindung zwischen dem Auslass 6 und einem Auslass 32 der Spritze 30. Der Inhalt der Spritze 30 kann nun mittels eines Vorschubglieds 33 durch den Auslass 32 und den Auslass 6 in das Gehäusevolumen 25 des Gehäusezylinders 5 verabreicht werden. Dabei wird das Austragglied 3 aus dem Gehäuse 1 heraus gedrückt, da das eingeführte Produkt gegen die Austragfläche. 18 drückt

Im gezeigten Beispiel ist bereits eine Komponente des fluiden Produkts innerhalb des Gehäusevolumens 25 des Gehäuses 1 der Austragvorrichtung gelagert Diese Komponente kann beispielsweise als Pulver vorliegen. Eine zweite Komponente wird aus der Spritze 30 in die Austragvorrichtung eingebracht, um gemeinsam mit der ersten Komponente das gewünschte fluide Produkt zu bilden. Sobald die zweite Komponente vollständig aus der Ladevorrichtung 30 in die Austragvorrichtung überführt wurde, kann das Kupplungsstück 31 vom Auslass 6 entfernt werden. Anschliessend wird die Kappe 4 über dem Auslass 6 angebracht, und es kann ein Mischvorgang wie zu Figur 3 beschrieben, innerhalb der Austragvorrichtung mittels der Mischeinrichtung durchgeführt werden.

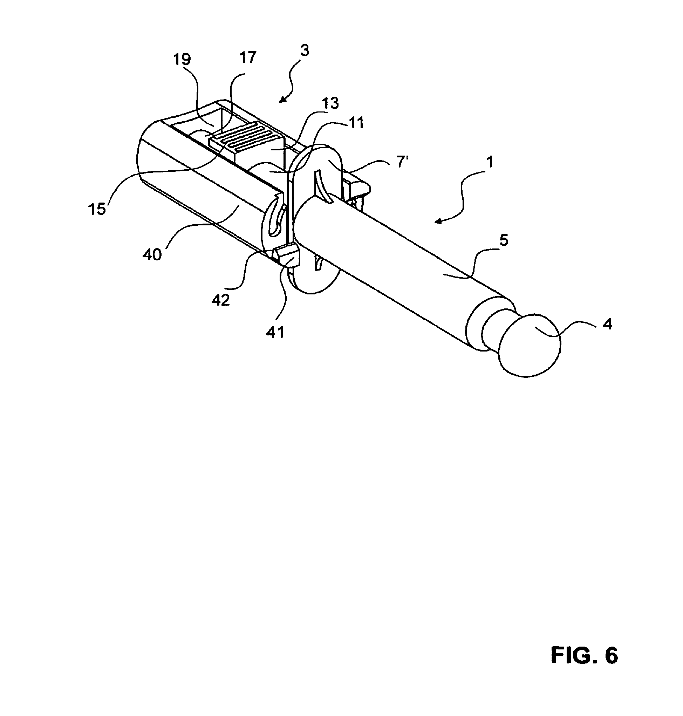

In Figur 6 ist eine zweite Ausführungsform einer Austragvorrichtung nach der vorliegenden Erfindung gezeigt. Bauteile die mit denjenigen der ersten Ausführungsform vergleichbar sind werden mit gleichen Bezugszeichen benannt. Die Austragvorrichtung umfasst ein Gehäuse 1 mit einem Gehäusezylinder 5, eine Mischeinrichtung mit einem Mischrohr 11 und ein Austragglied 3 mit einem Austragrohr 17. Am vorderen Ende des Gehäusezylinders 5 ist eine Abdeckkappe 4 angeordnet. Am hinteren Ende sind zwei Halte- bzw. Rastflügel 7' radial abstehend angeordnet. Das Mischrohr 11 kommt innerhalb des Gehäusezylinders 5 zu liegen und das Austragrohr 17 wird innerhalb des Mischrohrs 11 geführt. Am hinteren Ende des Austragrohrs 17 ist eine Druckplatte 19 vorgesehen, von der zwei Ausleger 40 zu beiden Seiten des Austragrohrs 17 parallel zum Austragrohr abstehen. Die Ausleger 40 flankieren somit das Austragrohr 17 zu beiden Seiten. Am hinteren Ende des Mischrohrs 11 ist ein Griffblock 13 vorgesehen, der zwischen den beiden Auslegern 40 zu liegen kommt. Die Seitenflächen des Mischrohrs 11 kommen dabei an den Innenflächen der Ausleger 40 zu liegen und können an diesen entlang gleiten. Die Ausleger 40 bilden somit eine Führung für den Griffblock 13, wenn das Mischrohr 11 innerhalb des Gehäusezylinders 5 relativ zum Austragglied verschoben wird. Der Griffblock 13 weist ferner Griffflächen 15 auf, welche geringfügig über die seitlichen Flächen der Ausleger 40 hervorstehen.

Zur Verschiebung des Austragglieds 3 innerhalb des Gehäusezylinders 5 wird der Gehäusezylinder 5 in eine Drehposition gebracht, in welcher die Rastflügel 7' zwischen den Auslegern 40 zu liegen kommen. In dieser Position kann das Austragrohr 17 in den Gehäusezylinder 5 eingeschoben werden, wobei die beiden Ausleger 40 an den beiden gegenüberliegenden Seiten des Gehäusezylinders 5 vorbei gleiten und die Rastflügel 7' zwischen sich aufnehmen. Die Ausleger 40 bilden somit eine Führungseinrichtung für das Aüstragglied 3 beim Vorschub des Austragrohrs 17 im Gehäuse 1.

Am vorderen Ende der Ausleger 40 sind an der Frontfläche in Längsrichtung der Ausleger abstehende Rasthaken 41 angeordnet. Die Rasthaken 41 sind derart ausgebildet, dass sie zwischen Frontfläche der Ausleger 40 und dem Haken eine Rastnut 42 ausbilden. Die Öffnung einer solchen Rastnut ist in Umfangsrichtung des Gehäusezylinders ausgerichtet. An den beiden gegenüberliegenden Auslegern 40 ist jeweils ein Rasthaken 41 punktsymmetrisch zur Längsachse des Gehäusezylinders vorgesehen. Demnach weisen die Öffnungen der Rastnuten in Umfangsrichtung in dieselbe Richtung.

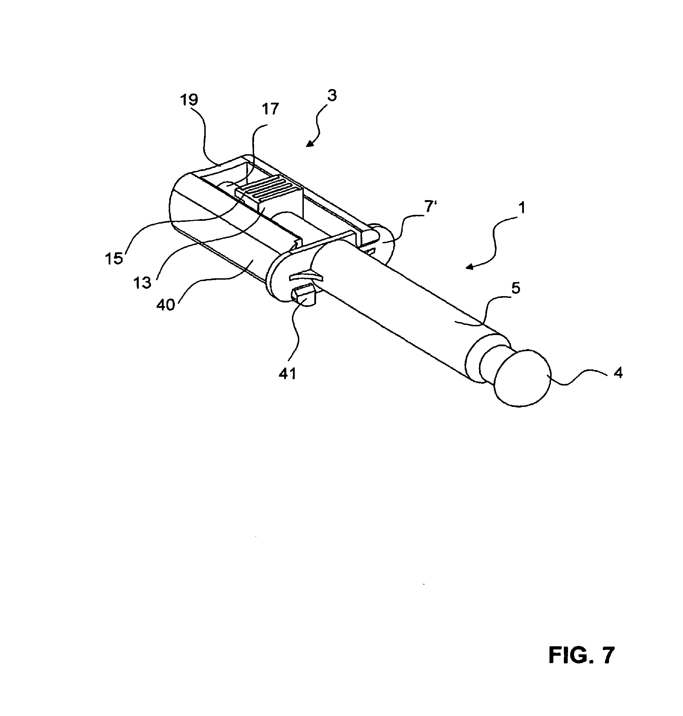

In Figur 7 ist die Austragvorrichtung in einer arretierten Position gezeigt, in welcher das Austragglied 3 relativ zum Gehäuse 1 arretiert ist. Hierfür wurde das Austragglied 3 derart weit aus dem Gehäusezylinder 5 herausgezogen, bis die Frontseite der Ausleger 40 hinter den Rastflügeln 7' zu liegen kommen. Anschliessend wird das Austragglied 3 relativ zum Gehäuse 1 derart verdreht, dass die Rastflügel 7' vor den Frontseiten der Ausleger 40 vorbei gleiten bis sie in die Rastnuten 42 an den Rasthaken 41 eingreifen. In dieser Arretierposition ist das Austragglied 3 gegenüber einer Längsbewegung relativ zum Gehäuse 1 arretiert.

Zum Mischen eines fluiden Produkts innerhalb des Gehäusezylinders 5 kann nun das Mischrohr 11 mittels dem Griffblock 13 in de Gehäusezylinder 5 vor und zurück bewegt werden. Hierfür wird der Griffblock 13 zwischen den Auslegern 40 von einem hinteren Anschlag an der Druckplatte 19 bis zu einem vorderen Anschlag an den Rastflügeln 7' hin- und herverschoben. Nach Beendigung des Mischvorgangs, kann das Austragglied 3 in Gegenrichtung relativ zum Gehäuse 1 verdreht werden, so dass die Rastflügel 7' aus den Rasthaken heraus gedreht werden. Die Rasthaken stehen vorzugsweise derart weit von der Frontseite der Ausleger 40 hervor, dass sie in dieser Drehrichtung einen Anschlag für die Rastflügel 7' bilden. Sobald die Rastflügel 7' an den Rasthaken anschlagen, kommen die Rastflügel 7' zwischen den Ausleger 40 zu liegen und das Austragrohr 17 kann durch Druck auf die Druckplatte 19 in den Gehäusezylinder 5 eingeschoben werden. Nachdem die Kappe 4 abgenommen wurde kann dadurch das gemischte fluide Produkt durch den Auslass am Gehäusezylinder 5 ausgetragen werden.

### BEZUGSZEICHENLISTE

| | | | |
|---|---|---|---|
| 1 | Gehäuses | 17 | Austragrohr |
| 2 | Mischeinrichtung | 18 | Austragfläche |
| 3 | Austragglied | 19 | Druckplatte |
| 4 | Abdeckkappe | 20 | Gleitarm |
| 5 | Gehäusezylinder | 21 | Rastarm |
| 6 | Auslass | 22 | Rastnocken |
| 7 | Halteflügel | 23 | Dichtlippe |
| 7' | Rastflügel | 24 | Boden |
| 8 | Leisten/Gleitkanal | 25 | Gehäusevolumen |
| 9 | Hülse | 30 | Ladevorrichtung |
| 10 | Restöffnung | 31 | Kupplungsstück |
| 11 | Mischrohr | 32 | Auslass |
| 12 | Mischelement | 33 | Vorschubglied |
| 13 | Griffblock | 40 | Ausleger |
| 14 | Dichtlippe | 41 | Rasthaken |
| 15 | Grifffläche | 42 | Rastnut |
| 16 | Gleitdurchgang | | |

## Patentansprüche

1. Austragvorrichtung zum Austragen eines fluiden Produkts, umfassend:
- ein Gehäuse (1) mit einem Auslass (6) an einem vorderen Gehäuseende,
- ein beweglich in dem Gehäuse gelagertes Austragglied (3), das mit seinem hinteren Ende aus einem hinteren Gehäuseende ragt,
- eine Mischeinrichtung (2) zum Mischen des fluiden Produkts innerhalb des Gehäuses,
wobei das fluide Produkt in einem zwischen dem Auslass (6) und dem Austragglied (3) ausgebildeten Gehäusevolumen (25) vorgesehen ist und durch Vorschub des Austragglieds (3) innerhalb des Gehäuses (1) von einer hinteren Stellung in eine vordere Stellung in Richtung des Auslasses (6) durch den Auslass aus dem Gehäuse (1) austragbar ist, **dadurch gekennzeichnet, dass** die Mischeinrichtung ein Mischrohr (11), das zwischen dem Gehäuse (1) und dem Austragglied (3) beweglich im Gehäuse (1) gelagert ist, und wenigstens ein Mischelement (12) aufweist, das innerhalb des Gehäusevolumens (25) am vorderen Ende des Mischrohres (11) angeordnet ist, und dass das Austragglied (3) zumindest teilweise innerhalb des Mischrohrs (11) angeordnet ist.

2. Austragvorrichtung nach Anspruch 1, **dadurch gekennzeichnet, dass** das Mischrohr (11) mit seinem hinteren Ende aus dem hinteren Gehäuseende hervorsteht.

3. Austragvorrichtung nach einem der vorhergehenden Ansprüche, **dadurch gekennzeichnet, dass** das Austragglied (3) aus dem hinteren Ende des Mischrohrs (11) hervorsteht.

4. Austragvorrichtung nach einem der vorhergehenden Ansprüche, **dadurch gekennzeichnet, dass** das Mischrohr (11) am hinteren, aus dem Gehäuse (1) hervorstehenden Ende einen Griffblock (13) aufweist, der zwischen dem hinteren Gehäuseende und dem hinteren Ende des Austragglieds (3) angeordnet ist.

5. Austragvorrichtung nach dem vorhergehenden Anspruch, **dadurch gekennzeichnet, dass** das Gehäuse (1) am hinteren Ende einen Gehäuseanschlag und das Austragglied (3) am hinteren Ende einen Stoppanschlag (19) aufweisen, zwischen welchen der Griffblock (13) des Mischrohrs (11) verschieblich ist.

6. Austragvorrichtung nach einem der vorhergehenden Ansprüche, **dadurch gekennzeichnet, dass** eine Aussenwand des Mischrohres (11) fluiddicht an einer Innenwand des Gehäuses (1) und das Austragglied (3) fluiddicht an der Innenwand des Mischrohres (11) gelagert ist.

7. Austragsvorrichtung nach einem der vorhergehenden Ansprüche, **dadurch gekennzeichnet, dass** das Mischrohr (11) relativ zum Gehäuse (1) und/oder zum Austragglied (3) drehbar gelagert ist.

8. Austragvorrichtung nach einem der vorhergehenden Ansprüche, **dadurch gekennzeichnet, dass** bei einer hinteren Stellung des Austragglieds (3) relativ zum Gehäuse das Mischrohr (11) innerhalb des Gehäusevolumens (25) zum Mischen des fluiden Produkts zwischen einer vorderen und einer hinteren Position beweglich ist, wobei das fluide Produkt in der vorderen Position innerhalb des Mischrohrs (11) aufgenommen ist.

9. Austragvorrichtung einem der vorhergehenden Ansprüche, **dadurch gekennzeichnet, dass** wenigstens ein Gleitarm (20) am Austragglied (3) und wenigstens ein Gleitkanal (8) am Gehäuse (1), in dem der Gleitarm (20) gleitend gelagert ist, vorgesehen ist.

10. Austragvorrichtung nach einem der vorhergehenden Ansprüche, **dadurch gekennzeichnet, dass** eine Arretiereinrichtung zur lösbaren Arretierung des Austragglieds (3) in einer hinteren Stellung am Gehäuse (1) vorgesehen ist.

11. Austragvorrichtung nach Anspruch 10, **dadurch gekennzeichnet, dass** an dem wenigstens einen Gleitarm (20) Rastmittel (21) vorgesehen sind, die mit Gegenrastmitteln (10) die am Gleitkanal (8) vorgesehen sind, lösbar verrasten.

12. Austragvorrichtung nach einem der vorhergehenden Ansprüche, **dadurch gekennzeichnet, dass** eine Blockiereinrichtung zur Blockierung einer Bewegung des Austragglieds (3) in der hinteren Stellung relativ zum Gehäuse (1) entgegen der Vorschubrichtung vorgesehen ist.

13. Austragvorrichtung nach einem der vorhergehenden Ansprüche, **dadurch gekennzeichnet, dass** eine Führungseinrichtung zur Führung des Griffblocks (13) des Mischrohrs (11) bei einer Verschiebung entlang dem Austragglied (3) vorgesehen ist.

14. Austragvorrichtung nach dem vorhergehenden Anspruch, **dadurch gekennzeichnet, dass** die Führungseinrichtung durch wenigstens einen parallel zum Austragglied (3) verlaufenden Gleitarm (20) oder Ausleger (40), der am Austragglied (3) angeordnet ist und parallel zu diesem verläuft, gegeben ist.

15. System aus einer Austragvorrichtung nach einem der Ansprüche 1 bis 14 und einer Ladevorrichtung (30), wobei im Gehäusevolumen (25) der Austragvorrichtung eine erste Mischkomponente untergebracht ist und aus der Ladevorrichtung (30) eine zweite Mischkomponente in das Gehäusevolumen (25) einbringbar ist.

## Claims

1. A discharge apparatus for discharging a fluid product, comprising:
- a housing (1) with an outlet (6) at a front housing end,
- a discharge member (3) which is mounted moveably in the housing and projects with its rear end out of a rear housing end,
- a mixing device (2) for mixing the fluid product inside the housing,
the fluid product being provided in a housing volume (25) formed between the outlet (6) and the discharge member (3) and being dischargeable out of the housing (1) through the outlet as a result of the advance of the discharge member (3) inside the housing (1) from a rear position into a front position in the direction of the outlet (6), **characterized in that** the mixing device has a mixing tube (11), which is mounted moveably in the housing (1) between the housing (1) and the discharge member (3), and at least one mixing element (12), which is arranged inside the housing volume (25) at the front end of the mixing tube (11), and that the discharge member (3) is arranged at least partially within the mixing tube (11).

2. The discharge apparatus as claimed in claim 1, **characterized in that** the mixing tube (11) projects with its rear end out of the rear housing end.

3. The discharge apparatus as claimed in one of the preceding claims, **characterized in that** the discharge member (3) projects out of the rear end of the mixing tube (11).

4. The discharge apparatus as claimed in one of the preceding claims, **characterized in that** the mixing tube (11) has, at the rear end projecting out of the housing (1), a grip block (13) which is arranged between the rear housing end and the rear end of the discharge member (3).

5. The discharge apparatus as claimed in the preceding claim, **characterized in that** the housing (1) has a housing abutment at the rear end and the discharge member (3) has a stop abutment (19) at the rear end, between which abutments the grip block (13) of the mixing tube (11) is displaceable.

6. The discharge apparatus as claimed in one of the preceding claims, **characterized in that** an outer wall of the mixing tube (11) is mounted, fluid-tight, on an inner wall of the housing (1), and the discharge member (3) is mounted, fluid-tight, on the inner wall of the mixing tube (11).

7. The discharge apparatus as claimed in one of the preceding claims, **characterized in that** the mixing tube (11) is mounted rotatably in relation to the housing (1) and/or the discharge member (3).

8. The discharge apparatus as claimed in one of the preceding claims, **characterized in that**, in a rear position of the discharge member (3) in relation to the housing, the mixing tube (11) is moveable between a front and a rear position within the housing volume (25) for the purpose of mixing the fluid product, the fluid product being received within the mixing tube (11) in the front position.

9. The discharge apparatus as claimed in one of the preceding claims, **characterized in that** at least one sliding arm (20) is provided on the discharge member (3), and at least one sliding channel (8), in which the sliding arm (20) is mounted slideably, is provided on the housing (1).

10. The discharge apparatus as claimed in one of the preceding claims, **characterized in that** a detaining device for the releasable detention of the discharge member (3) in a rear position is provided on the housing (1).

11. The discharge apparatus as claimed in claim 10, **characterized in that** latching means (21) are provided on the at least one sliding arm (20) and releaseably latch together with counter latching means (10) which are provided in the sliding channel (8).

12. The discharge apparatus as claimed in one of the preceding claims, **characterized in that** a blocking device for blocking movement of the discharge member (3) in the rear position in relation to the housing (1) opposite to the direction of advance is provided.

13. The discharge apparatus as claimed in one of the preceding claims, **characterized in that** a guide device for guiding the grip block (13) of the mixing tube (11) during displacement along the discharge member (3) is provided.

14. The discharge apparatus as claimed in the preceding claim, **characterized in that** the guide device is formed by at least one sliding arm (20) or outrigger (40) which runs parallel to the discharge member (3) and which is arranged on the discharge member (3) and runs parallel to the latter.

15. A system composed of a discharge apparatus as claimed in one of claims 1 to 14 and of a loading apparatus (30), a first mix component being accommodated in the housing volume (25) of the discharge apparatus, and a second mix component being introducible into the housing volume (25) from the loading apparatus (30).

## Revendications

1. Dispositif dispenseur d'un produit fluide, comprenant :
- un logement (1) avec une sortie (6) à une extrémité avant du logement,
- un élément dispenseur (3) disposé mobile dans le logement (1), lequel fait saillie, avec son extrémité arrière, d'une extrémité arrière du logement,
- un dispositif mélangeur (2) pour mélanger le produit fluide à l'intérieur du logement,
sachant que le produit fluide est prévu dans un volume de logement (25) formé entre la sortie (6) et l'élément dispenseur (3) et, par poussée de l'élément dispenseur (3) à l'intérieur du logement (1) d'une position arrière à une position avant en direction de la sortie (6), peut être dispensé hors du logement (1) à travers la sortie, **caractérisé en ce que** le dispositif mélangeur présente un tube mélangeur (11) disposé mobile dans le logement (1), entre le logement (1) et l'élément dispenseur (3), et au moins un élément mélangeur (12) disposé à l'intérieur du volume de logement (25) à l'extrémité avant du tube mélangeur (11), et que l'élément dispenseur (3) est disposé au moins partiellement à l'intérieur du tube mélangeur (11).

2. Dispositif dispenseur selon la revendication 1, **caractérisé en ce que** le tube mélangeur (11) fait saillie, avec son extrémité arrière, de l'extrémité arrière du logement.

3. Dispositif dispenseur selon la revendication 1 ou 2, **caractérisé en ce que** l'élément dispenseur (3) fait saillie de l'extrémité arrière du tube mélangeur (11).

4. Dispositif dispenseur selon l'une des revendications précédentes, **caractérisé en ce que** le tube mélangeur (11) présente à l'extrémité arrière en saillie du logement (1), un bloc de préhension (13) qui est disposé entre l'extrémité arrière du logement et l'extrémité arrière de l'élément dispenseur (3).

5. Dispositif dispenseur selon la revendication précédente, **caractérisé en ce que** le logement (1) présente une butée de logement à l'extrémité arrière et l'élément dispenseur (3) présente une butée d'arrêt (19) à l'extrémité arrière, entre lesquelles le bloc de préhension (13) du tube mélangeur (11) peut être déplacé.

6. Dispositif dispenseur selon l'une des revendications précédentes, **caractérisé en ce qu'**une paroi extérieure du tube mélangeur (11) est disposée en étanchéité fluidique à une paroi intérieure du logement (1) et l'élément dispenseur (3) est disposé en étanchéité fluidique à la paroi intérieure du tube mélangeur (11).

7. Dispositif dispenseur selon l'une des revendications précédentes, **caractérisé en ce que** le tube mélangeur (11) est disposé en étant rotatif par rapport au logement (1) et/ou à l'élément dispenseur (3).

8. Dispositif dispenseur selon l'une des revendications précédentes, **caractérisé en ce que** dans une position arrière de l'élément dispenseur (3) par rapport au logement, le tube mélangeur (11) est mobile entre une position avant et une position arrière à l'intérieur du volume de logement (25) pour mélanger le produit fluide, sachant que le produit fluide est reçu à l'intérieur du tube mélangeur (11) dans la position avant.

9. Dispositif dispenseur selon l'une des revendications précédentes, **caractérisé en ce qu'**au moins un bras coulissant (20) est prévu à l'élément dispenseur (3) et au moins un canal coulissant (8) est prévu au logement (1), dans lequel le bras coulissant (20) est disposé en pouvant coulisser.

10. Dispositif dispenseur selon l'une des revendications précédentes, **caractérisé en ce qu'**un dispositif de blocage est prévu pour le blocage séparable de l'élément dispenseur (3) dans une position arrière au logement (1).

11. Dispositif dispenseur selon la revendication 10, **caractérisé en ce que** des moyens d'encliquetage (21) sont prévus à l'au moins un bras coulissant (20), lesquels s'encliquent de manière séparable avec des moyens de contre-encliquetage (10) prévus au canal coulissant (8).

12. Dispositif dispenseur selon l'une des revendications précédentes, **caractérisé en ce qu'**un dispositif d'arrêt est prévu pour l'arrêt d'un mouvement de l'élément dispenseur (3) dans la position arrière par rapport au logement (1) contre le sens d'avancée.

13. Dispositif dispenseur selon l'une des revendications précédentes, **caractérisé en ce qu'**un dispositif de guidage est prévu pour guider le bloc de préhension (13) du tube mélangeur (11) dans un mouvement le long de l'élément dispenseur (3).

14. Dispositif dispenseur selon la revendication précédente, **caractérisé en ce que** le dispositif de guidage est fourni par au moins un bras coulissant (20) parallèle à l'élément dispenseur (3) ou une potence (40) disposée à l'élément dispenseur (3) et parallèle à celui-ci.

15. Système d'un dispositif dispenseur selon l'une des revendications 1 à 14 et d'un dispositif de chargement (30), dans lequel une première composante du mélange est logée dans le volume de logement (25) du dispositif dispenseur et une seconde composante du mélange peut être déposée du dispositif de chargement (30) dans le volume de logement (25).
